# EUROPÄISCHE PATENTSCHRIFT

(11) **EP 1 341 805 B1**
(45) Veröffentlichungstag und Bekanntmachung des Hinweises auf die Patenterteilung: **19.05.2004**
(21) Anmeldenummer: 01993609.5
(22) Anmeldetag: 09.11.2001
(51) Int. Cl.: C07J 41/00

(54) **VERFAHREN ZUR HERSTELLUNG VON 4-(17-ALPHA-ALKOXYMETHYL-17-BETA-SUBSTITUIERTEN-3-OXOESTRA-4,9-DIEN-11-BETA-YL)BENZALDEHYD-(1E)-OXIM-DERIVATEN**
METHOD FOR THE PRODUCTION OF 4-(17-ALPHA-ALKOXYMETHYL-17-BETA-SUBSTITUTED 3-OXOESTRA-4,9-DIEN-11-BETA-YL)BENZALDEHYD-(1E)-OXIME DERIVATIVES
PROCEDE DE PRODUCTION DE DERIVES DE 3-OXOESTRA-4,9-DIEN-11-BETA-YL)BENZALDEHYD-(1E)-OXIMES SUBSTITUEES PAR 4-(17-ALPHA- ALKOXYMETHYL-17-BETA

(30) Priorität: 10.11.2000 DE 10056677
(43) Veröffentlichungstag der Anmeldung: 10.09.2003
(73) Patentinhaber: Schering AG, 13353 Berlin-Wedding (DE)
(72) Erfinder: SCHUBERT, Gerd, 07743 Jena (DE); RING, Sven, 07749 Jena (DE)
(74) Vertreter: Ziebig, Marlene, Dr. Dipl.-Chem.
(86) Internationale Anmeldenummer: PCT/DE2001/004219
(87) Internationale Veröffentlichungsnummer: WO 2002/038583

(56) Entgegenhaltungen:
- EP-A- 0 648 779
- DD-A- 61 263
- DE-A- 1 618 340
- JUMAR, A.; HELD, P.; SCHULZE, W.: "Über die Herstellung und Reaktionsfähigkeit von Chlorformyloximen" Z. CHEM., Bd. 7, Nr. 9, 1967, Seiten 344-345, XP001068343

## Beschreibung

Die Erfindung betrifft ein Verfahren zur Herstellung von 4-(17α-Alkoxymethyl-17β-substituierten-3-oxoestra-4,9-dien-11β-yl)benzaldehyd-(1E)-oxim-Derivaten der allgemeinen Formel (I) 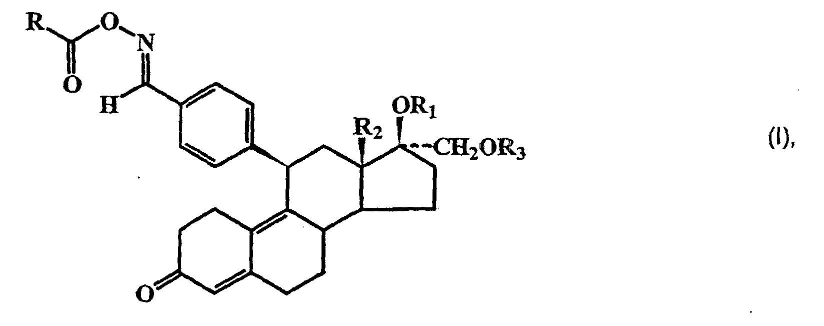 worin R eine Aminogrupe, einen O-C₁₋₇-Alkyl- oder O-Arylrest, einen S-C₁₋₇-Alkyl- oder S-Arylrest, einen NH-C₁₋₇-Alkyl- oder NH-Arylrest oder einen N-Di-C₁₋₇-alkylrest bedeutet, R₁ ein Wasserstoffatom oder einen C₁₋₆-Alkylrest bedeutet, R₂ einen C₁₋₄-Alkylrest bedeutet und R₃ einen C₁₋₆-Alkylrest bedeutet.
4-(17α-Alkoxymethyl-17β-substituierte-3-oxoestra-4,9-dien-11β-yl)benzaldehyd-(1E)oxim-Derivate sind bekannt. Substanzen dieser Art sind in DE 4332283 A1 (EP 0 648 778 B1) oder DE 43 32 284 A1 beschrieben. Die Verbindungen sind wegen der günstigen antigestagenen und geringen antiglucocorticoiden Wirkung von allgemeinem Interesse für die Behandlung einer Reihe von hormonabhängigen Erkrankungen der Frau, wie beispielsweise der Endometriose.

Das bisherige Verfahren zu ihrer Herstellung geht von einem Oxim der allgemeinen Formel (II) 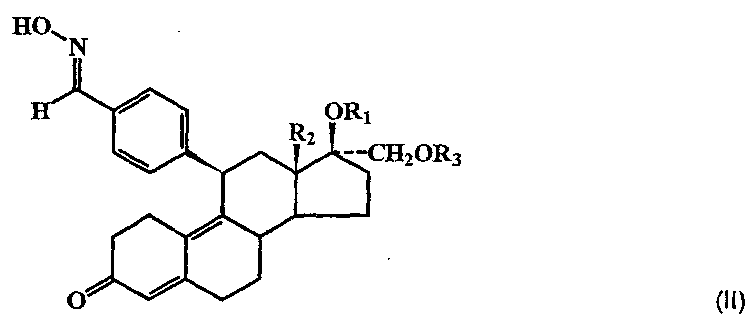 aus,
worin R₁, R₂ und R₃ die vorstehend gegebene Bedeutung haben können. Durch Umsetzung mit Chlorkohlensäureestern, Chlorkohlensäurethiolestern oder Isocyanaten läßt sich die Hydroxylgruppe des Oxims verestern bzw. in die Urethane überführen. Nachteil des Verfahrens ist die Bildung von Nitrilen durch Wassereliminierung und die teilweise Reaktion an der freien 17β-Hydroxylgruppe mit dem Veresterungsreagenz. Die Nebenprodukte lassen sich chromatographisch oft schlecht abtrennen.

Die Aufgabe der vorliegenden Erfindung ist deshalb, ein technisch einfaches und effektives Verfahren zur Herstellung von 4-(17α-Alkoxymethyl-17β-substituierten-3-oxoestra-4,9-dien-11β-yl)benzaldehyd-(1E)oxim-Derivaten der Formel (I) zur Verfügung zu stellen, das einerseits den Angriff des Veresterungsreagenzes am C-17 und andererseits die Eliminierung von Wasser aus den Benzaldoxim zum Nitrilderivat verhindert und damit die Zielverbindungen der Formel (I) mit höherer Ausbeute und Selektivität liefert.

Erfindungsgemäß wird diese Aufgabe dadurch gelöst, daß Benzaldoxime der allgemeinen Formel (II), worin R₁, R₂ und R₃ die vorstehend gegebene Bedeutung haben, mit Chlorameisensäuretrichlormethylester oder Phosgen in einem inerten Lösungsmittel in Gegenwart von tert.-Aminen, vorzugsweise Triethylamin, bei Temperaturen zwischen -35 und +30°C zu den Chlorkohlensäure-Derivaten der Formel (III) 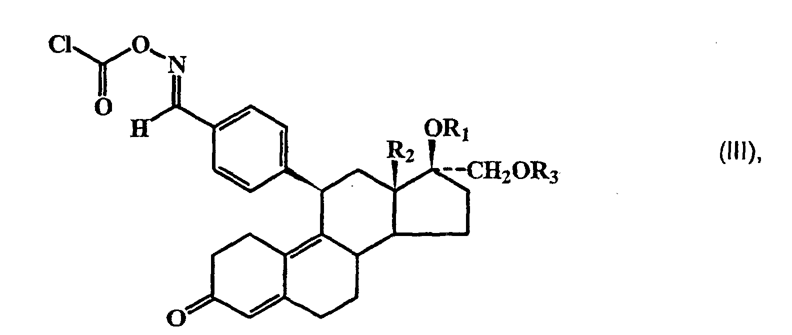 umgesetzt werden, in der R₁, R₂ und R₃ die vorstehend gegebene Bedeutung haben, und die Chlorkohlensäure-Derivate der Formel (III) mit C₁₋₇-Alkyl- oder Arylalkoholen, C₁₋₇-Alkyl- oder Arylthioalkoholen oder Ammoniak oder C₁₋₇-Alkyl- oder Arylaminen oder Di-C₁₋₇-alkylaminen zu den 4-(17α-Alkoxymethyl-17β-substituierten-3-oxoestra-4,9-dien-11β-yl)benzaldehyd-(1E)oxim-Derivaten der Formel (I) umgesetzt werden.

Überraschenderweise reagiert der Chlorameisensäuretrichlormethylester weder mit einer freien 17β-Hydroxylgruppe noch wird unter den gewählten Bedingungen eine Eliminierung von Wasser aus der Benzaldoximgruppe beobachtet.

Hierdurch ergibt sich im Vergleich zu dem Verfahren des Standes der Technik eine höhere Ausbeute. So läßt sich beispielsweise die Phenylurethanverbindung gemäß Beispiel 11 mit bis zu 30 % höherer Ausbeute oder der Kohlensäureester gemäß Beispiel 1 mit 10 % besserer Ausbeute herstellen, als die bisher mit dem Verfahren gemäß des Standes der Technik möglich war. Ein weiterer Vorteil des erfindungsgemäßen Verfahrens im Vergleich mit dem Verfahren des Standes der Technik ist dessen größere Variationsbreite. Es können praktisch alle Alkohole, Amine oder Thiolverbindungen mit dem Chlorkohlensäureester (III) umgesetzt werden.

Bevorzugte Ausführungsformen der Erfindung sind in den Unteransprüchen angegeben. Wegen weiterer Vorteile der Erfindung wird auf die folgende Beschreibung und die Ausführungsbeispiele verwiesen.

Die Umsetzung erfolgt in einem inerten Lösungsmittel oder in einem Gemisch aus inerten Lösungsmitteln, wobei aromatische Lösungsmittel, wie Toluol, oder Ether, wie Tetrahydrofuran (THF) oder Methyl-tert.-butylether, bevorzugt sind.

Die Umsetzung wird in Gegenwart eines tertiären Amins, bevorzugt Triethylamin und Pyridin, bei Temperaturen zwischen -35 und +30°C, bevorzugt -35 und +30°C, besonders bevorzugt -35°C ausgeführt.

Die Isolierung und Reinigung der Verbindungen der Formel (I) erfolgt nach üblichen Verfahren, wie Umkristallisation oder Chromatographie, beispielsweise präparative Schichtchromatographie.

Unter "Alkylrest" wird in der vorliegenden Erfindung ein verzweigter oder geradkettiger Alkylrest oder ein cyclischer Alkylrest verstanden, der im Ring ein oder zwei Heteroatome aufweisen kann, die aus einem Stickstoffatom, einem Sauerstoffatom und einem Schwefelatom ausgewählt werden können.

Als C₁₋₄-, C₁₋₆- bzw. C₁₋₇-Alkylreste seien beispielsweise Methyl-, Ethyl, n-Propyl, i-Propyl, n-Butyl, i-Butyl oder tert.-Butyl, n-Pentyl oder i-Pentyl, n-Hexyl-, 2-Pentyl-, 3-Pentyl-, 2,3-Dimethylbutyl-, n-Heptyl-, 2-Methylhexyl-, 3-Methylhexyl-, 2,2-Dimethylpentyl-, 3,3-Dimethylpentyl-, 2,3-Dimethylpentyl- und 2,2,3-Trimethylbutylgruppen genannt. Bevorzugt bedeuten R₁, R₂ und R₃ einen C₁₋₃-Alkylrest, besonders bevorzugt eine Methylgruppe.

R bedeutet einen O-C₁₋₇-Alkyl-, O-Arylrest, S-C₁₋₇-Alkyl-, S-Arylrest, NH-C₁₋₇-Alkyl-, NH-Arylrest bzw. N-Di-C₁₋₇-alkylrest oder eine Aminogruppe

Im Falle von R ist auch ein cyclischer Alkylrest bevorzugt, der im Ring ein oder zwei Heteroatome aufweisen kann, die aus einem Stickstoffatom, einem Sauerstoffatom und einem Schwefelatom ausgewählt werden können, wie beispielsweise eine Tetrahydropyranylgruppe.

Unter dem Begriff "Aryl" wird in der vorliegenden Anmeldung ein Aryl-, Aralkyl- oder Alkylarylrest mit 1 bis 10 Kohlenstoffatomen verstanden. Beispiele für einen Phenylrest sind eine Phenylgruppe, eine Halogenphenylgruppe, eine Nitrophenylgruppe oder eine Naphtylgruppe. Beispiele für einen Aralkylrest sind eine Toluenylgruppe (Methylphenylgruppe), Halogentoluenylgruppe, Ethylphenylgruppe, Dimethylphenylgruppe oder Trimethylphenylgruppe. Beispiele für einen Alkylarylrest sind eine freie oder aromatisch substituierte Benzylgruppe, wie eine Benzylgruppe oder Halogenbenzylgruppe. Unter "Halogen" wird ein Fluor-, Chlor-, Brom- oder lodatom verstanden.

Bevorzugt bedeutet R einen O-C₁₋₄-Alkyl-, O-Arylrest, S-C₁₋₄-Alkyl-, S-Arylrest, NH₂- NH-C₁₋₄-Alkyl-, NH-Arylrest bzw. N-Di-C₁₋₄-alkylrest, wobei eine Amino-, Methoxy-, Ethoxy-, Phenoxy-, Methylthio-, Ethylthio-, NH-Methyl-, NH-Ethyl-, NH-Propyl-, NH-Phenyl- oder NH-Tetrahydropyranylgruppe besonders bevorzugt sind.

Am stärksten bevorzugt sind im Rahmen von Verbindungen der Formel (I) die folgenden Verbindungen:
4-[17β-Methoxy-17α-(methoxymethyl)-3-oxoestra-4,9-dien-11β-yl]benzaldehyd-1E-[O-(ethyloxy)carbonyl]oxim,
4-[17β-Hydroxy-17α-(methoxymethyl)-3-oxoestra-4,9-dien-11β-yl]benzaldehyd-1E-[O-(ethyloxy)carbonyl]oxim,
4-[17β-Methoxy-17α-(methoxymethyl)-3-oxoestra-4,9-dien-11β-yl]benzaldehyd-1E-[O-(methoxy)carbonyl]oxim,
4-[17β-Hydroxy-17α-(methoxymethyl)-3-oxoestra-4,9-dien-11β-yl]benzaldehyd-1E-[O-(methoxy)carbonyl]oxim,
4-[17β-Methoxy-17α-(methoxymethyl)-3-oxoestra-4,9-dien-11β-yl]benzaldehyd-1E-[O-(ethylamino)carbonyl]oxim,
4-[17β-Hydroxy-17α-(methoxymethyl)-3-oxoestra-4,9-dien-11β-yl]benzaldehyd-1E-[O-(ethylamino)carbonyl]oxim,
4-[17β-Methoxy-17α-(methoxymethyl)-3-oxoestra-4,9-dien-11β-yl]benzaldehyd-1E-[O-(ethylthio)carbonyl]oxim,
4-[17β-Hydroxy-17α-(methoxymethyl)-3-oxoestra-4,9-dien-11β-yl]benzaldehyd-1E-[O-(ethylthio)carbonyl]oxim),
4-[17β-Methoxy-17α-(methoxymethyl)-3-oxoestra-4,9-dien-11β-yl]benzaldehyd-1E-[O-(methylthio)carbonyl]oxim,
4-[17β-Methoxy-17α-(meth-oxymethyl)-3-oxoestra-4,9-dien-11β-yl]benzaldehyd-1E-[O-(phenyloxy)carbonyl]oxim,
4-[17β-Methoxy-17α-(methoxymethyl)-3-oxoestra-4,9-dien-11β-yl]benzaldehyd-1E-[O-(phenylamino)carbonyl]oxim,
4-[17β-Methoxy-17α-(methoxymethyl)-3-oxoestra-4,9-dien-11β-yl]benzaldehyd-1E-[O-(tetrahydropyranylamino)carbonyl]oxim,
4-[17β-Methoxy-17α-(methoxymethyl)-3-oxoestra-4,9-dien-11β-yl]benzaldehyd-1E-[O-(amino)carbonyl]oxim und
4-[17β-Methoxy-17α-(methoxymethyl)-3-oxoestra-4,9-dien-11β-yl]benzaldehyd-1E-[O-(propylamino)carbonyl]oxim.

Bei den Ausgangsverbindungen der Formel (II) handelt es sich um bekannte Verbindungen, die beispielsweise nach den in DE 4332283 A1 (EP 0 648 778 B1) bzw. DE 43 32 284 A1 beschriebenen Verfahren oder nach den in den Anmeldungen "Verfahren zur Herstellung von 4-(17α-substituierten-3-oxoestra-4,9-dien-11β-yl)benzaldehyd-(1E oder 1Z)-oximen" und "Verfahren zur Herstellung von 4-(17α-Methyl-substituierten 3-Oxoestra-4,9-dien-11β-yl)behzaldehyd-(1E oder 1Z)-oximen" beschriebenen Verfahren, die am gleichen Tag wie die vorliegende Anmeldung von der Anmelderin der vorliegenden Anmeldung beim Deutschen Patent- und Markenamt eingereicht wurden, hergestellt werden können.

Die Verbindungen werden gut am Gestagenrezeptor gebunden, zeigen im Tierexperiment eine starke antigestagene Aktivität und besitzen nur eine geringe glucocorticoide Rezeptorbindung DE 4332283 A1 (EP 0 648 778 B1) oder DE 43 32 284 A1.

Die nachfolgenden Beispiele dienen zur näheren Beschreibung der Erfindung.

Allgemeine Vorschrift zur Herstellung von 4-[17β-Methoxy-(17α-(methoxymethyl)-3-oxoestra-4,9-dien-11β-yl]benzaldehyd-1E-(chlorcarbonyl)oxim (III)
900 mg 4-[17β-substituiertes-17α-(Methoxymethyl)-3-oxoestra-4,9-dien-11β-yl]benzaldehyd-1E-oxim werden in 100 ml Lösungsmittel (THF, Toluol oder Methyl-tert.-butylether) gelöst und bei -35°C mit 0,37 ml Chlorameisensäuretrimethylester in 20 ml Lösungsmittel versetzt. Man rührt 30 Minuten bei dieser Temperatur, leitet 20 Minuten einen Argonstrom hindurch und erhält (III) als Zwischenprodukt.

### Beispiel 1

### 4-[17β-Methoxy-(17α-(methoxymethyl)-3-oxoestra-4,9-dien-11β-yl]benzaldehyd-1E-[O-(ethoxy)carbonyl]oxim

Zu einer Lösung von 1 g frisch hergestelltem 4-[17β-Methoxy-(17α-(methoxymethyl)-3oxoestra-4,9-dien-11β-yl]benzaldehyd-1E-(chlorcarbonyl)oxim (IIIa) in 100 ml Toluol wird bei -35°C ein Gemisch aus 20 ml Ethanol und 20 ml Toluol zugetropft. Man läßt auf Raumtemperatur erwärmen, engt im Vakuum auf die Hälfte ein, gibt Wasser zu und trennt die Phasen. Die organische Phase wird mit Wasser gewaschen, mit Natriumsulfat getrocknet und im Vakuum verdampft. Das Rohprodukt wird in Aceton/Hexan umkristallisiert.
Schmp. 137 bis 148°C (Aceton/n-Hexan); α_{D} = +204° (CHCl₃); ¹H-NMR: 8,30 (s, 1H, HC=N), 7,63 (d, 2H, J = 8,1, H-3'), 7,25 (d, 2H, J = 8,1, H-2'), 5,78 (s, 1H, H-4), 4,35 (d, 1H, J = 7,2, H-11), 3,56 (d, 2H, J = 10,8, CH₂O), 3,41 (s, 3H, OCH₃), 3,40 (d, 2H, J = 10,8 , CH₂O), 3,25 (s, 3H, OCH₃), 1,38 (t, 3H, J= 7,0, CH₂CH₃), ,51 (s, 3H, H-18).

### Beispiel 2

### 4-[17β-Hydroxy-(17α-(methoxymethyl)-3-oxoestra-4,9-dien-11β-yl]benzaldehyd-1E-[O-(ethoxy)carbonyl]oxim

Zu 1g 4-[17β-Hydroxy-(17α-(methoxymethyl)-3-oxoestra-4,9-dien-11β-yl]benzaldehyd-1E-(chlorcarbonyl)oxim (IIIb) in 100 ml THF werden bei -35°C 20 ml Ethanol zugetropft. Man gießt in Eiswasser ein, extrahiert mit Essigester, wäscht neutral, trocknet mit Natriumsulfat, filtriert ab und engt im Vakuum ein. Das Rohprodukt wird in Aceton/Methyl-tert.-butylether umkristallisiert.
Schmp. 161 bis 171°C (Aceton/Methyl-tert.-butylether); α_{D} = +201° (CHCl₃); ¹H-NMR: 8,31 (s, 1H, HC=N), 7,64 (d, 2H, J = 8,4, H-3'), 7,25 (d, 2H, J = 8,4, H-2'), 5,79 (s, 1H, H-4), 4,38 (d, 1H, J = 7,2, H-11), 4,35 (q, 2H, CH₂CH₃), 3,56 (d, 2H, J = 9,0, CH₂O), 3,41 (s, 3H, OCH₃), 3,21 (d, 2H, J = 9,0, CH₂O), 2,65 (s, 1H, OH), 1,38 (t, 3H, J= 7,0, CH₂CH₃), 0,52 (s, 3H, H-18).

### Beispiel 3

### 4-[17β-Methoxy-(17α-(methoxymethyl)-3-oxoestra-4,9-dien-11β-yl]benzaldehyd-1E-[O-(methoxy)carbonyl]oxim

Zu 1g (IIIa) in 100 ml THF werden bei -35°C 15 ml Methanol zugetropft. Man gießt in Eiswasser ein, extrahiert mit Essigester, wäscht neutral, trocknet die Lösung mit Natriumsulfat, filtriert ab und engt im Vakuum ein. Das Rohprodukt wird in Methyl-tert.-butylether umkristallisiert.
Schmp. 110 bis 123°C (Methyl-tert.-butylether); α_{D} = +171° (CHCl₃); ¹H-NMR: 8,31 (s, 1H, HC=N), 7,63 (d, 2H, J = 8,1, H-3'), 7,26 (d, 2H, J = 9,0, H-2'), 5,78 (s, 1H, H-4), 4,40 (d, 1H, J = 7,2, H-11), 3,92 (s, 3H, OCH₃), 3,55 (d, 2H, J = 10,5, CH₂O), 3,41 (s, 3H, OCH₃), 3,40 (d, 2H, J = 10,8 , CH₂O), 3,25 (s, 3H, OCH₃), 0,51 (s, 3H, H-18).

### Beispiel 4

### 4-[17β-Hydroxy-(17α-(methoxymethyl)-3-oxoestra-4,9-dien-11β-yl]benzaldehyd-1E-[O-(methoxy)carbonyl]oxim

Zu 1g (IIIb) in 100 ml THF werden bei -35°C 20 ml Methanol zugetropft. Man gießt in Eiswasser ein, extrahiert mit Essigester, wäscht neutral, trocknet mit Natriumsulfat, filtriert ab und engt im Vakuum ein. Das Rohprodukt wird durch präparative Schichtchromatographie an Kieselgel-gereinigt und in Aceton/Methyltert.-butylether umkristallisiert.
Schmp. 158 bis 163°C (Aceton/Methyl-tert.-butylether); α_{D} = +203° (CHCl₃); ¹H-NMR: 8,32 (s, 1H, HC=N), 7,64 (d, 2H, J = 8,4, H-3'), 7,24 (d, 2H, J = 8,4, H-2'), 5,79 (s, 1H, H-4), 4,41 (d, 1H, J = 7,2, H-11), 3,93 (s, 3H, OCH₃), 3,56 (d, 2H, J = 9,0, CH₂O), 3,41 (s, 3H, OCH₃), 3,21 (d, 2H, J = 9,3, CH₂O), 2,66 (s,1H, OH), 0,51 (s, 3H, H-18).

### Beispiel 5

### 4-[17β-Methoxy-(17α-(methoxymethyl)-3-oxoestra-4,9-dien-11β-yl]benzaldehyd-1E-[O-(ethytamino)carbonyl)oxim

Zu 1g (IIIa) in 100 ml THF werden bei -35°C 30 ml einer Lösung von 10 ml Ethylamin in 20 ml THF zugetropft. Man gießt in Eiswasser ein, extrahiert mit Methyl-tert.-butylether, wäscht neutral, trocknet über Natriumsulfat, filtriert ab und engt im Vakuum ein. Das Rohprodukt wird durch präparative Schichtchromatographie an Kieselgel gereinigt und in Aceton/ n-Hexan umkristallisiert.
Schmp.167 bis 171°C (Zersetzung, Aceton/n-Hexan); α_{D} = +221° (CHCl₃); ¹H-NMR: 8,29 (s, 1H, HC=NOR), 7,58 (d, 2H, J = 8,4, H-3'), 7,27 (d, 2H, J = 8,4, H-2'), 6,27 (t,1H, J=5,7, NH),5,79 (s, 1H, H-4), 4,41 (d, 1H, J = 7,3, H-11), 3,56 (d, 2H, J = 10,6, CH₂O), 3,42 (d, 2H, J = 10,6 CH₂O), 3,41 (s, 3H, OCH₃), 3,25 (s, 3H, OCH₃), 1,24 (t, 3H, J= 7,2, CH₂CH₃),0,52 (s, 3H, H-18).

### Beispiel 6

### 4-[17β-Hydroxy-(17α-(methoxymethyl)-3-oxoestra-4,9-dien-11β-yl]benzaldehyd-1E-[O-(ethylamino)carbonyl)oxim

Zu 1g (IIIb) in 100 ml Methyl-tert.-butylether werden bei -35°C 30 ml einer Lösung von 10 ml Ethylamin in Methyl-tert.-butylether zugetropft. Man gießt in Eiswasser ein, trennt die Phasen, wäscht neutral, trocknet die organische Phase mit Natriumsulfat, filtriert ab und engt im Vakuum ein. Das Rohprodukt wird in Aceton/Methyl-tert.butylether umkristallisiert.
Schmp. 188 bis 191 °C (Aceton/Methyl-tert.-butylether); α_{D} = +228° (CHCl₃); ¹H-NMR: 8,30 (s, 1H, HC=NOR), 7,59 (d, 2H, J = 8,4, H-3'), 7,28 (d, 2H, J = 8,4, H-2'), 6,25 (t,1H, J=5,2, NH), 5,79 (s, 1H, H-4), 4,42 (d, 1H, J = 7,2, H-11), 3,56 (d, 2H, J = 9,0, CH₂O), 3,42 (s, 3H, OCH₃), 3,37 (q, 7,3, NHCH₂CH₃), 3,22 (d, 2H, J =9,0, CH₂O), 1,23 (t, 3H, J= 7,3, NHCH₂CH₃), 0,53 (s, 3H, H-18).

### Beispiel 7

### 4-[17β-Methoxy-(17α-(methoxymethyl)-3-oxoestra-4,9-dien-11β-yl]benzaldehyd-1E-[O-(ethylthio)carbonyl)oxim

Zu 1g (IIIa) in 100 ml Methyl-tert.-butylether werden bei -35°C 20 ml einer Lösung von 4 ml Ethylmercaptan in 16 ml Methyl-tert.-butylether zugetropft. Man gießt in Eiswasser ein, trennt die Phasen, wäscht neutral, trocknet die organische Phase mit Natriumsulfat, filtriert ab und engt im Vakuum ein. Das Rohprodukt wird in Aceton/n-Hexan umkristallisiert.
Schmp. 148 bis 155°C (Aceton/n-Hexan); α_{D} = +235° (CHCl₃); ¹H-NMR: 8,31 (s, 1H, HC=NOR), 7,61 (d, 2H, J = 8,4, H-3'), 7,27 (d, 2H, J = 8,1, H-2'), 5,79 (s, 1H, H-4), 4,41 (d, 1H, J = 7,2, H-11), 3,57 (d, 2H, J = 10,8, CH₂O), 3,42 (d, 2H, J = 10,8, CH₂O), 3,41 (s, 3H, OCH₃), 3,25 (s, 3H, OCH₃), 2,95 (q, 2H, J=4,5 und 15, SCH₂CH₃), 1,37 (t, 3H, J= 7,0, SCH₂CH₃),0,52 (s, 3H, H-18).

### Beispiel 8

### 4-[17β-Hydroxy-(17α-(methoxymethyl)-3-oxoestra-4,9-dien-11β-yl]benzaldehyd-1E-[O-(ethylthio)carbonyl)oxim

Zu 1g (IIIb) in 100 ml Methyl-tert.-butylether werden bei -35°C 10 ml einer Lösung von 3 ml Ethylmercaptan in 7 ml Methyl-tert.-butylether zugetropft. Man gießt in Eiswasser ein, trennt die Phasen, wäscht neutral, trocknet die organische Phase mit Natriumsulfat, filtriert ab und engt im Vakuum ein. Das Rohprodukt wird durch präparative Schichtchromatographie an Kieselgel gereinigt und in Dichlormethan/Essigester umkristallisiert.
Schmp. 176 bis 180°C (Dichlormethan/Essigester); α_{D} = +226° (CHCl₃); ¹H-NMR: 8,32 (s, 1H, HC=NOR), 7,62 (d, 2H, J = 8,2, H-3'), 7,27 (d, 2H, J = 8,2, H-2'), 5,79 (s, 1H, H-4), 4,41 (d, 1H, J = 6,7, H-11), 3,56 (d, 2H, J = 9,2, CH₂O), 3,42 (s, 3H, OCH₃), 3,21 (d, 2H, J = 9,2, CH₂O), 2,95 (q, 2H, SCH₂CH₃), 1,36 (t, 3H, J= 7,2, SCH₂CH₃), 0,52 (s, 3H, H-18).

### Beispiel 9

### 4-[17β-Methoxy-(17α-(methoxymethyl)-3-oxoestra-4,9-dien-11β-yl]benzaldehyd-1 E-[O-(methylthio)carbonyl)oxim

In 10 ml gekühltes THF wird 5 Minuten Methylmercaptan eingeleitet und diese Lösung wird bei -35°C zu 1g (IIIa) in 100 ml THF getropft. Nach 30 Minuten wird in Eiswasser gegossen, die Phasen werden getrennt, die organische Phase mit Wasser gewaschen, mit Natriumsulfat getrocknet, filtriert und im Vakuum eingeengt. Das Rohprodukt wird in Aceton/n-Hexan umkristallisiert.
Schmp. 134 bis 137°C (Aceton/Methyl-tert.-butylether); α_{D} = +184° (CHCl₃); ¹H-NMR: 8,32 (s, 1H, HC=NOR), 7,61 (d, 2H, J = 7,8, H-3'), 7,29 (d, 2H, J = 7,8, H-2'), 5,78 (s, 1H, H-4), 4,41 (d, 1H, J = 7,2, H-11), 3,57 (d, 2H, J = 10,5, CH₂O), 3,42 (d, 2H, J = 10,8, CH₂O), 3,40 (s, 3H, OCH₃), 3,25 (s, 3H, OCH₃), 2,40 (s, 3H, SCH₃); 0,52 (s, 3H, H-18).

### Beispiel 10

### 4-[17β-Hydroxy-(17α-(methoxymethyl)-3-oxoestra-4,9-dien-11β-yl]benzaldehyd-1E-[O-(phenyloxy)carbonyl]oxim

Die Herstellung erfolgt analog Beispiel 3.
Das Rohprodukt wird in Aceton/n-Hexan umkristallisiert.
Schmp. 101 bis 106°C (Zers.); α_{D} = +179° (CHCl₃); ¹H-NMR: 8,41 (s, 1H, HC=NOR), 7,66 (d, 2H, J = 8,4, H-3'), 7,41 (d, 2H, J = 7,5, H-2'), 7,25-7,46 (m, 5H, Aromat), 5,79 (s, 1H, H-4), 4,41 (d, 1H, J = 7,2, H-11), 3,57 (d, 2H, J = 10,5, CH₂O), 3,42 (d, 2H, J = 10,8, CH₂O), 3,41 (s, 3H, OCH₃), 3,25 (s, 3H, OCH₃), 0,52 (s, 3H, H-18).

### Beispiel 11

### 4-[17β-Hydroxy-(17α-(methoxymethyl)-3-oxoestra-4,9-dien-11β-yl]benzaldehyd-1 E-[O-(phenylamino)carbonyl]oxim

Die Herstellung erfolgt analog Beispiel 5.
Das Rohprodukt wird in Aceton umkristallisiert.
Schmp. 241 bis 246°C; α_{D} = +178° (CHCl₃); ¹H-NMR: 8,41 (s, 1H, HC=NOR), 7,56 (d, 2H, J = 8,1, H-3'), 7,29 (d, 2H, J = 8,4, H-2'), 7,26-7,32 (m, 5H, Aromat), 5,79 (s, 1H, H-4), 5,17 (s, 1H, NH), 4,41 (d, 1H, J = 6,6, H-11), 3,53 (d, 2H, J = 10,8, CH₂O), 3,42 (d, 2H, J = 10,8, CH₂O), 3,41 (s, 3H, OCH₃), 3,25 (s, 3H, OCH₃), 0,47 (s, 3H, H-18).

### Beispiel 12

### 4-[17β-Hydroxy-(17α-(methoxymethyl)-3-oxoestra-4,9-dien-11β-yl]benzaldehyd-1E-[O-(amino)carbonyl]oxim

Die Herstellung erfolgt analog Beispiel 5.
Das Rohprodukt wird in Methanol /Methyl-tert.-butylether umkristallisiert.
Schmp. 145 bis 153°C (Zers.); α_{D} = +213° (CHCl₃); ¹H-NMR (DMSO): 8,49 (s, 1H, HC=NOR), 7,72 (d, 2H, J = 7,8, H-3'), 7,32 (d, 2H, J = 7,5, H-2'), 7,11(s, 2H, NH₂), 5,68 (s, 1H, H-4), 4,45 (d, 1H, J = 6,9, H-11), 3,57 (d, 2H, J = 11,1, CH₂O), 3,38 (d, 2H, J = 11,1, CH₂O), 3,29 (s, 3H, OCH₃), 3,12 (s, 3H, OCH₃), 0,42 (s, 3H, H-18).

### Beispiel 13

### 4-[17β-Hydroxy-(17α-(methoxymethyl)-3-oxoestra-4,9-dien-11β-yl]benzaldehyd-1E-[O-(tetrahydropyranylamino)carbonyl]oxim

Die Herstellung erfolgt analog Beispiel 5.
Farbloser Schaum; α_{D} = +181° (CHCl₃); ¹H-NMR: 8,30 (s, 1H, HC=NOR), 7,57 (d, 2H, J = 8,4, H-3'), 7,27 (d, 2H, J = 8,1, H-2'), 6,72 (s, 1H, NH), 5,79 (s, 1H, H-4), 5,05 (t, 1H, THP), 4,41 (d, 1H, J = 7,2, H-11), 3,56 (d, 2H, J = 10,5, CH₂O), 3,42 (d, 2H, J = 10,5, CH₂O), 3,41 (s, 3H, OCH₃), 3,25 (s, 3H, OCH₃), 0,52 (s, 3H, H-18).

### Beispiel 14

### 4-[17β-Hydroxy-(17α-(methoxymethyl)-3-oxoestra-4,9-dien-11β-yl]benzaldehyd-1E-[O-(propylamino)carbonyl]oxim

Die Herstellung erfolgt analog Beispiel 5.
Das Rohprodukt wird in Aceton / n-Hexan umkristallisiert.
Schmp. 145 °C (Zers.); α_{D} = +222° (CHCl₃); ¹H-NMR (CDCl₃): 8,30 (s, 1H, HC=NOR), 7,57 (d, 2H, J = 7,8, H-3'), 7,27 (d, 2H, J = 7,5, H-2'), 6,28 (t, 1H, NH), 5,79 (s, 1H, H-4), 4,41 (d, 1H, J = 6,9, H-11), 3,55 (d, 2H, J = 10,5, CH₂O), 3,43 (d, 2H, J = 10,5, CH₂O), 3,41 (s, 3H, OCH₃), 3,30 (q, 2H, Propyl), 3,25 (s, 3H, OCH₃), 0,97 (t, 3H, CH₃), 0,52 (s, 3H, H-18).

## Patentansprüche

1. Verfahren zur Herstellung von 4-(17α-Alkoxymethyl-17β-substituierten-3-oxoestra-4,9-dien-11β-yl)benzaidehyd-(1E)-oxim-Derivaten der allgemeinen Formel (I) 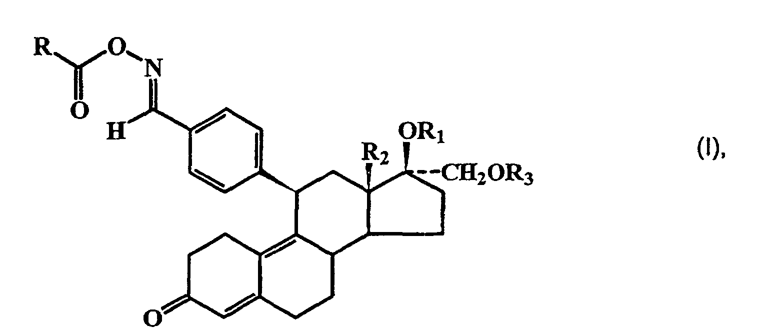 worin R eine Aminogrupe, einen O-C₁₋₇-Alkyl- oder O-Arylrest, einen S-C₁₋₇-Alkyl- oder S-Arylrest, einen NH-C₁₋₇-Alkyl- oder NH-Arylrest oder einen N-Di-C₁₋₇-alkylrest bedeutet, R₁ ein Wasserstoffatom oder einen C₁₋₆-Alkylrest bedeutet, R₂ einen C₁₋₄-Alkylrest bedeutet und R₃ einen C₁₋₆-Alkylrest bedeutet, **dadurch gekennzeichnet, daß** Benzaldoxime der allgemeinen Formel (II) 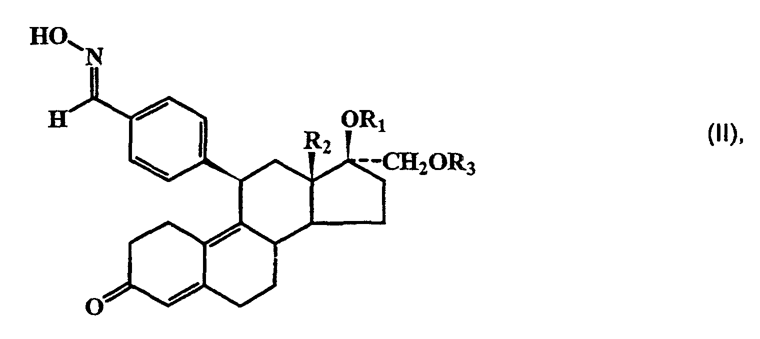 worin R₁, R₂ und R₃ die vorstehend gegebene Bedeutung haben, mit Chlorameisensäuretrichlormethylester oder Phosgen in einem inerten Lösungsmittel in Gegenwart von tert.-Aminen, vorzugsweise Triethylamin, bei Temperaturen zwischen -35 und +30°C zu den Chlorkohlensäure-Derivaten der Formel (III) umgesetzt werden, 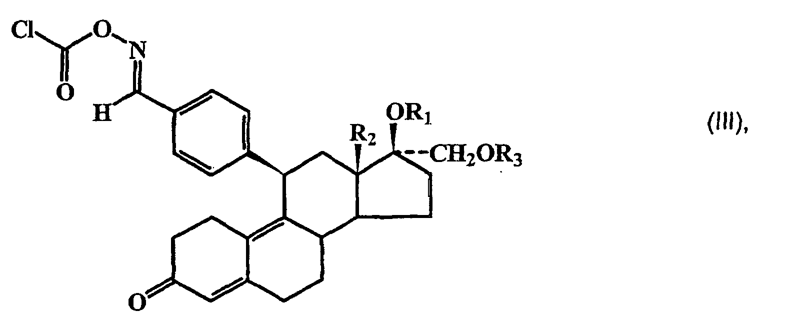 in der R₁, R₂ und R₃ die vorstehend gegebene Bedeutung haben, und
die Chlorkohlensäure-Derivate der Formel (III) mit C₁₋₇-Alkyl- oder Arylalkoholen, C₁₋₇-Alkyl- oder Arylthioalkoholen oder C₁₋₇-Alkyl- oder Arylaminen oder Di-C₁₋₇-alkylaminen zu den 4-(17α-Alkoxymethyl-17β-substituierten-3-oxoestra-4,9-dien-11β-yl)benzaldehyd-(1E)oxim-Derivaten der Formel (I) umgesetzt werden.

2. Verfahren nach Anspruch 1, wobei R₁ einen C₁₋₃-Alkylrest, vorzugsweise eine Methylgruppe bedeutet.

3. Verfahren nach einem der Ansprüche 1 oder 2, wobei R₂ einen C₁₋₃-Alkylrest, vorzugsweise eine Methylgruppe bedeutet.

4. Verfahren nach einem der Ansprüche 1 bis 3, wobei R₃ einen C₁₋₃-Alkylrest, vorzugsweise eine Methylgruppe bedeutet.

5. Verfahren nach einem der Ansprüche 1 bis 4, wobei R eine Amino-, Methoxy-, Ethoxy-, Phenoxy-, Methylthio-, Ethylthio-, NH-Methyl-, NH-Ethyl-, NH-Propyl-, NH-Phenyl- oder NH-Tetrahydropyranylgruppe bedeutet.

6. Verfahren nach einem der Ansprüche 1 bis 5, **dadurch gekennzeichnet, daß** folgende Verbindungen der Formel (I) hergestellt werden:
4-[17β-Methoxy-17α-(methoxymethyl)-3-oxoestra-4,9-dien-11β-yl]benzaldehyd-1E-[O-(ethyloxy)carbonyl]oxim,
4-[17β-Hydroxy-17α-(methoxymethyl)-3-oxoestra-4,9-dien-11β-yl]benzaldehyd-1E-[O-(ethyloxy)carbonyl]oxim,
4-[17β-Methoxy-17α-(methoxymethyl)-3-oxoestra-4,9-dien-11β-yl]benzaldehyd-1E-[O-(methoxy)carbonyl]oxim,
4-[17β-Hydroxy-17α-(methoxymethyl)-3-oxoestra-4,9-dien-11β-yl]benzaldehyd-1E-[O-(methoxy)carbonyl]oxim,
4-[17β-Methoxy-17α-(methoxymethyl)-3-oxoestra-4,9-dien-11β-yl]benzaldehyd-1E-[O-(ethylamino)carbonyl]oxim,
4-[17β-Hydroxy-17α-(methoxymethyl)-3-oxoestra-4,9-dien-11β-yl]benzaldehyd-1E-[O-(ethylamino)carbonyl]oxim,
4-[17β-Methoxy-17α-(methoxymethyl)-3-oxoestra-4,9-dien-11β-yl]benzaldehyd-1E-[O-(ethylthio)carbonyl]oxim,
4-[17β-Hydroxy-17α-(methoxymethyl)-3-oxoestra-4,9-dien-11β-yl]benzaldehyd-1E-[O-(ethylthio)carbonyl]oxim),
4-[17β-Methoxy-17α-(methoxymethyl)-3-oxoestra-4,9-dien-11β-yl]benzaldehyd-1E-[O-(methylthio)carbonyl]oxim,
4-[17β-Methoxy-17α-(meth-oxymethyl)-3-oxoestra-4,9-dien-11β-yl]benzaldehyd-1E-[O-(phenyloxy)carbonyl]oxim,
4-[17β-Methoxy-17α-(methoxymethyl)-3-oxoestra-4,9-dien-11β-yl]benzaldehyd-1E-[O-(phenylamino)carbonyl]oxim,
4-[17β-Methoxy-17α-(methoxymethyl)-3-oxoestra-4,9-dien-11β-yl]benzaldehyd-1E-[O-(tetrahydropyranylamino)carbonyl]oxim,
4-[17β-Methoxy-17α-(methoxymethyl)-3-oxoestra-4,9-dien-11β-yl]benzaldehyd-1E-[O-(amino)carbonyl]oxim und
4-[17β-Methoxy-17α-(methoxymethyl)-3-oxoestra-4,9-dien-11β-yl]benzaldehyd-1E-[O-(propylamino)carbonyl]oxim.

## Claims

1. Process for the production of 4-(17α-alkoxymethyl-17β-substituted-3-oxoestra-4,9-dien-11β-yl)benzaldehyde-(1E)-oxime derivatives of general formula (I) 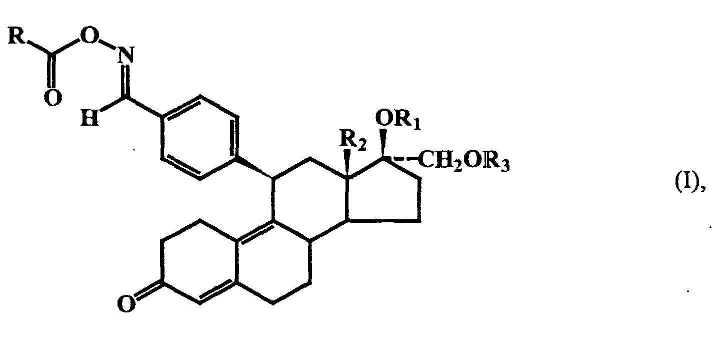 in which R means an amino group, an O-C₁₋₇-alkyl- or O-aryl radical, an S-C₁₋₇-alkyl- or S-aryl radical, an NH-C₁₋₇-alkyl- or NH-aryl radical or an N-di-C₁₋₇-alkyl radical, R₁ means a hydrogen atom or a C₁₋₆-alkyl radical, R₂ means a C₁₋₄-alkyl radical, and R₃ means a C₁₋₆-alkyl radical, **characterized in that** benzaldoximes of general formula (II) 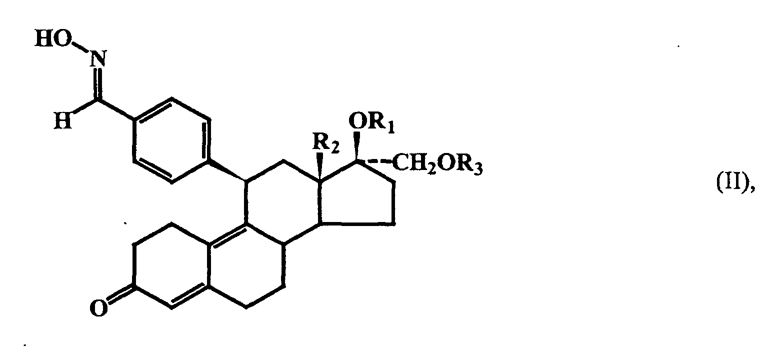 in which R₁, R₂ and R₃ have the meaning provided above, are reacted with chloroformic acid trichloromethyl ester or phosgene in an inert solvent in the presence of tert-amines, preferably triethylamine, at temperatures of between -35 and +30°C to form the chloroformic acid derivatives of formula (III), 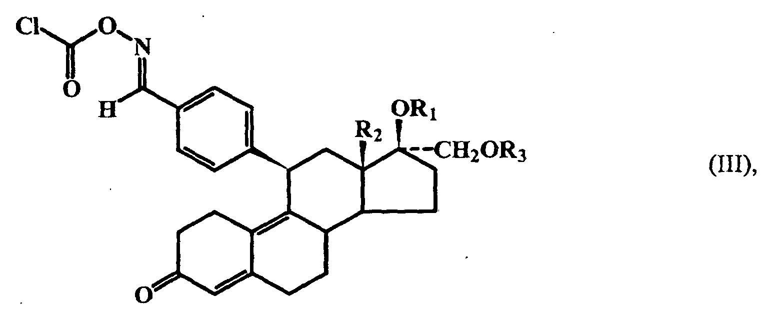 in which R₁, R₂ and R₃ have the meaning provided above, and the chloroformic acid derivatives of formula (III) are reacted with C₁₋₇-alkyl- or aryl alcohols, C₁₋₇-alkyl- or aryl thioalcohols or C₁₋₇-alkyl- or arylamines or di-C₁₋₇-alkylamines to form the 4-(17α-alkoxymethyl-17β-substituted-3-oxoestra-4,9-dien-11β-yl)benzaldehyde-(1E)oxime derivatives of formula (I).

2. Process according to claim 1, whereby R₁ means a C₁₋₃-alkyl radical, preferably a methyl group.

3. Process according to one of claims 1 or 2, whereby R₂ means a C₁₋₃-alkyl radical, preferably a methyl group.

4. Process according to one of claims 1 to 3, whereby R₃ means a C₁₋₃-alkyl radical, preferably a methyl group.

5. Process according to one of claims 1 to 4, whereby R means an amino-, methoxy-, ethoxy-, phenoxy-, methylthio-, ethylthio-, NH-methyl-, NH-ethyl-, NH-propyl-, NH-phenyl- or NH-tetrahydropyranyl group.

6. Process according to one of claims 1 to 5, wherein the following compounds of formula (I) are produced:
4-[17β-Methoxy-17α-(methoxymethyl)-3-oxoestra-4,9-dien-11β-yl]benzaldehyde-1E-[O-(ethyloxy)carbonyl]oxime,
4-[17β-Hydroxy-17α-(methoxymethyl)-3-oxoestra-4,9-dien-11β-yl]benzaldehyde-1E-[O-(ethyloxy)carbonyl]oxime,
4-[17β-Methoxy-17α-(methoxymethyl)-3-oxoestra-4,9-dien-11β-yl]benzaldehyde-1E-[O-(methoxy)carbonyl]oxime,
4-[17β-Hydroxy-17α-(methoxymethyl)-3-oxoestra-4,9-dien-11β-yl]benzaldehyde-1E-[O-(methoxy)carbonyl]oxime,
4-[17β-Methoxy-17α-(methoxymethyl)-3-oxoestra-4,9-dien-11β-yl]benzaldehyde-1E-[O-(ethylamino)carbonyl]oxime,
4-[17β-Hydroxy-17α-(methoxymethyl)-3-oxoestra-4,9-dien-11β-yl]benzaldehyde-1E-[O-(ethylamino)carbonyl]oxime,
4-[17β-Methoxy-17α-(methoxymethyl)-3-oxoestra-4,9-dien-11β-yl]benzaldehyde-1E-[O-(ethylthio)carbonyl]oxime,
4-[17β-Hydroxy-17α-(methoxymethyl)-3-oxoestra-4,9-dien-11β-yl]benzaldehyde-1E-[O-(ethylthio)carbonyl]oxime),
4-[17β-Methoxy-17α-(methoxymethyl)-3-oxoestra-4,9-dien-11β-yl]benzaldehyde-1E-[O-(methylthio)carbonyl]oxime,
4-[17β-Methoxy-17α-(methoxymethyl)-3-oxoestra-4,9-dien-11β-yl]benzaldehyde-1E-[O-(phenyloxy)carbonyl]oxime,
4-[17β-Methoxy-17α-(methoxymethyl)-3-oxoestra-4,9-dien-11β-yl]benzaldehyde-1E-[O-(phenylamino)carbonyl]oxime,
4-[17β-Methoxy-17α-(methoxymethyl)-3-oxoestra-4,9-dien-11β-yl]benzaldehyde-1E-[O-(tetrahydropyranylamino)carbonyl]oxime,
4-[17β-Methoxy-17α-(methoxymethyl)-3-oxoestra-4,9-dien-11β-yl]benzaldehyde-1E-[O-(amino)carbonyl]oxime and
4-[17β-Methoxy-17α-(methoxymethyl)-3-oxoestra-4,9-dien-11β-yl]benzaldehyde-1E-[O-(propylamino)carbonyl]oxime.

## Revendications

1. Procédé de préparation de dérivés de 4-(17α-alkoxyméthyl-17β-substitué-3-oxoestra-4,9-dién-11β-yl)benzaldéhyde-(1E)-oxime de formule générale (I) 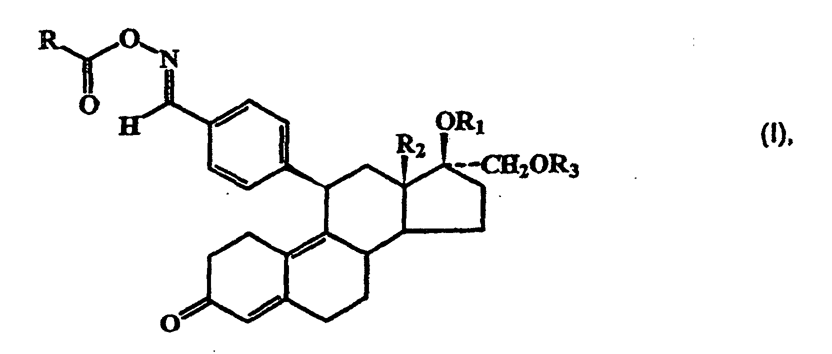 dans laquelle R représente un groupe amino, un radical O-alkyle en C₁₋₇ ou O-aryle, un radical S-alkyle en C₁₋₇ ou S-aryle, un radical NH-alkyle en C₁₋₇ ou NH-aryle, ou un radical N-dialkyle en C₁₋₇, R₁ représente un atome d'hydrogène ou un radical alkyle en C₁₋₆, R₂ représente un radical alkyle en C₁₋₄ et R₃ représente un radical alkyle en C₁₋₆, **caractérisé en ce que** des benzaldoximes de formule générale (II) 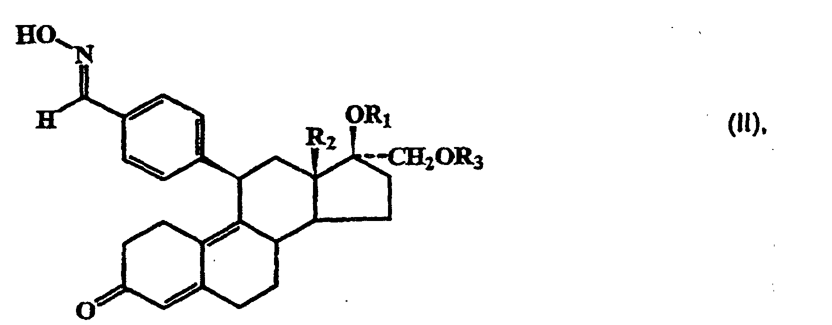 dans laquelle R₁, R₂ et R₃ ont la signification indiquée précédemment, sont convertis, avec du trichlorométhylester d'acide chloroformique ou du phosgène dans un solvant inerte, en présence de tert. amines, de préférence de triéthylamine, à des températures de - 35 à + 30° C, en dérivés d'acide chlorocarbonique de formule (III), 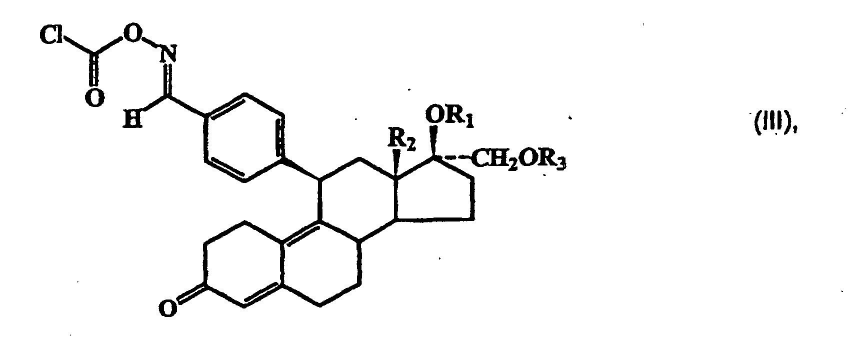 dans laquelle R₁, R₂ et R₃ ont la signification indiquée précédemment, et les dérivés d'acide chlorocarbonique de formule (III) sont convertis, avec des alkyl(C₁₋₇)- ou arylalcools, des alkyl(C₁₋₇)- ou arylthioalcools, ou des alkyl(C₁₋₇)- ou arylamines, ou des dialkyl(C₁₋₇)amines en dérivés de 4-(17α-alkoxyméthyl-17β-substitué-3-oxoestra-4,9-dién-11β-yl)benzaldéhyde-(1E)-oxime de formule (I).

2. Procédé selon la revendication 1, dans lequel R₁ représente un radical alkyle en C₁₋₃, de préférence un groupe méthyle.

3. Procédé selon une des revendications 1 ou 2, dans lequel R₂ représente un radical alkyle en C₁₋₃, de préférence un groupe méthyle.

4. Procédé selon une des revendications 1 à 3, dans lequel R₃ représente un radical alkyle en C₁₋₃, de préférence un groupe méthyle.

5. Procédé selon une des revendications 1 à 4, dans lequel R représente un groupe amino, méthoxy, éthoxy, phénoxy, méthylthio, éthylthio, NH-méthyle, NH-éthyle, NH-propyle, NH-phényle ou NH-tétrahydropyranyle.

6. Procédé selon une des revendications 1 à 5, **caractérisé en ce qu'**on prépare les composés suivants de formule (I) :
4-[17β-méthoxy-17α-(méthoxyméthyl)-3-oxoestra-4,9-dién-11β-yl]benzaldéhyde-1E-[O-(éthyloxy)-carbonyl]oxime,
4-[17β-hydroxy-17α-(méthoxyméthyl)-3-oxoestra-4,9-dién-11β-yl]benzaldéhyde-1E-[-[O-(éthyloxy)-carbonyl]oxime,
4-[17β-méthoxy-17α-(méthoxyméthyl)-3-oxoestra-4,9-dién-11β-yl]benzaldéhyde-1E-[O-(méthoxy)-carbonyl]oxime,
4-[17β-hydroxy-17α-(méthoxyméthyl)-3-oxoestra-4,9-dién-11β-yl]benzaldéhyde-1E-[O-(méthoxy)-carbonyl]oxime,
4-[17β-méthoxy-17α-(méthoxyméthyl)-3-oxoestra-4,9-dién-11β-yl]benzaldéhyde-1E-[O-(éthylamino)-carbonyl]oxime,
4-[17β-hydroxy-17α-(méthoxyméthyl)-3-oxoestra-4,9-dién-11β-yl]benzaldéhyde-1E-[O-(éthylamino)-carbonyl]oxime,
4-[17β-méthoxy-17α-(méthoxyméthyl)-3-oxoestra-4,9-dién-11β-yl]benzaldéhyde-1E-[O-(éthylthio)-carbonyl]oxime,
4-[17β-hydroxy-17α-(méthoxyméthyl)-3-oxoestra-4,9-dién-11β-yl]benzaldéhyde-1E-[O-(éthylthio)-carbonyl]oxime,
4-[17β-méthoxy-17α-(méthoxyméthyl)-3-oxoestra-4,9-dién-11β-yl]benzaldéhyde-1E-[O-(méthylthio)-carbonyl]oxime,
4-[17β-méthoxy-17α-(méthoxyméthyl)-3-oxoestra-4,9-dién-11β-yl]benzaldéhyde-1E-[O-(phényloxy)-carbonyl]oxime,
4-[17β-méthoxy-17α-(méthoxyméthyl)-3-oxoestra-4,9-dién-11β-yl]benzaldéhyde-1E-[O-(phénylamino)-carbonyl]oxime,
4-[17β-méthoxy-17α-(méthoxyméthyl)-3-oxoestra-4,9-dién-11β-yl]benzaldéhyde-1E-[O-(tétrahydropyranylamino)-carbonyl]oxime,
4-[17β-méthoxy-17α-(méthoxyméthyl)-3-oxoestra-4,9-dién-11β-yl]benzaldéhyde-1E-[O-(amino)-carbonyl]oxime, et
4-[17β-méthoxy-17α-(méthoxyméthyl)-3-oxoestra-4,9-dién-11β-yl]benzaldéhyde-1E-[O-(propylamino)-carbonyl]oxime.
